# EUROPEAN PATENT APPLICATION

(11) **EP 1 997 504 A1**
(43) Date of publication of application: **03.12.2008**
(21) Application number: 07715291.6
(22) Date of filing: 01.03.2007
(51) Int. Cl.: A61K 38/00, A61P 9/10

(54) **NOVEL ANTITHROMBOTIC AGENT**

(30) Priority: 01.03.2006 US 778489 P
(71) Applicant: Keio University, Tokyo 108-8345 (JP)
(72) Inventor: SUEMATSU, Makoto, Tokyo 160-8582 (JP); MANDAI, Shintaro, Yokohama-shi, Kanagawa 230-0012 (JP)
(74) Representative: Woods, Geoffrey Corlett
(86) International application number: PCT/JP2007/054489
(87) International publication number: WO 2007/102562

(57) **Abstract**

The present invention provides: a pharmaceutical composition comprising an albumin conjugated with polyethylene glycol; and a method of treating a human thrombosis or a human ischemic disease, comprising intravenously administering an albumin conjugated with polyethylene glycol to a patient.

## Description

### TECHNICAL FIELD

The present invention relates to an anti-thrombotic reagent, and a method of treating an ischemic disease such as myocardial infarction, cerebral infarction or occlusive arterial disease.

### BACKGROUND OF THE INVENTION

Until now, a variety of anti-thrombotic reagents have been available. These reagents are subdivided into two categories. That is, those acting on platelets and those modulating function of circulating coagulants. Reagents acting on platelets include those inhibiting cyclooxygenase (aspirin), those inhibiting phosphodiesterase (cilostazol), and those suppressing purinergic receptors on the cell membrane (thiazolidine derivatives). On the other hand, reagents controlling the action of coagulant factors include those inhibiting thrombin (anti-thrombin III), those inhibiting other coagulant factors (warfarin), and those activating fibrinolytic activities (tissue plasminogen activator; tPA and urokinase). These reagents have been used widely in the clinical field to treat patients with acute coronary insufficiency, stroke and cerebral ischemia.

However, these anti-thrombotic reagents have many problems, and thus an anti-thrombotic reagent having a novel action mechanism is required to treat such ischemic disease.

### DISCLOSURE OF THE INVENTION

Under such circumstances, a novel pharmaceutical composition that can be used in an anti-thrombotic treatment, the treatment of an ischemic disease or the like is desired.

As a result of intensive studies, the present inventors have found that an albumin conjugated with polyethylene glycol (PEG) has a function of controlling blood coagulation and/or platelet aggregation in a vascular injury area, and that the albumin conjugated with polyethylene glycol exhibits an anti-thrombotic effect, thereby completing the present invention.
(1) A pharmaceutical composition comprising an albumin conjugated with polyethylene glycol.
(2) The composition according to (1) above, which is an anti-thrombotic reagent or a reagent for treating an ischemic disease.
(3) The composition according to (1) or (2) above, wherein the above-described albumin is a human albumin.
(4) The composition according to (1) or (2) above, wherein the above-described albumin is a recombinant human serum albumin.
(5) A method of treating a thrombosis or an ischemic disease, comprising intravascularly administering an albumin conjugated with polyethylene glycol to a patient.
(6) The method according to (5) above, which treats a human.
(7) The method according to (5) or (6) above, wherein the above-described intravascular administration is an intravenous administration.
(8) The method according to any one of (5) to (7) above, wherein the above-described albumin is a human albumin.
(9) The method according to any one of (5) to (7) above, wherein the above-described albumin is a recombinant human serum albumin.
(10) Use of an albumin conjugated with polyethylene glycol for production of a pharmaceutical composition used in a treatment of an ischemic disease
(11) Use of an albumin conjugated with polyethylene glycol for production of an anti-thrombotic pharmaceutical composition.

As described above, the present invention provides a novel pharmaceutical composition. In a preferred embodiment of the present invention, the pharmaceutical composition can be used as an anti-thrombotic reagent or a reagent for treating an ischemic disease, and it has a function of controlling blood coagulation and/or platelet aggregation in a vascular injury area and the like and exhibits an anti-thrombotic effect and the like.

### BRIEF DESCRIPTION OF THE DRAWINGS

Figure 1(A) shows a calibration line for determination of PEG(10,000), and Figure 1(B) is a view showing a representative chart indicating PEG-rHSA and free PEG.
Figure 2 is a view showing the relationship between the viscosities and shear rates *in vitro* in PEG-rHSA or free rHSA. "○": Rat blood (whole blood samples from fasted rats); "□": rHSA; "•": PEG-rHSA conjugate; "△": free PEG + rHSA mixture.
Figure 3 is a view showing effects of administration of PEG-rHSA and rHSA on mean arterial blood pressure (MAP) before and after the 30%-isovolemic blood exchange in rats. Position change was carried out to optimize the condition of rats for determination of bleeding time by tail cut.
Figure 4(A) is a view showing alterations in bleeding time of rats by treatment with rHSA, PEG-rHSA and PEG+rHSA. **P*<0.05 (comparison of a control group with a PEG-rHSA-treated group). Figure 4(B) shows differences in hematocrit values, and Figure 4(C) shows differences in platelet counts. **P*<0.05 (comparison with the control group).

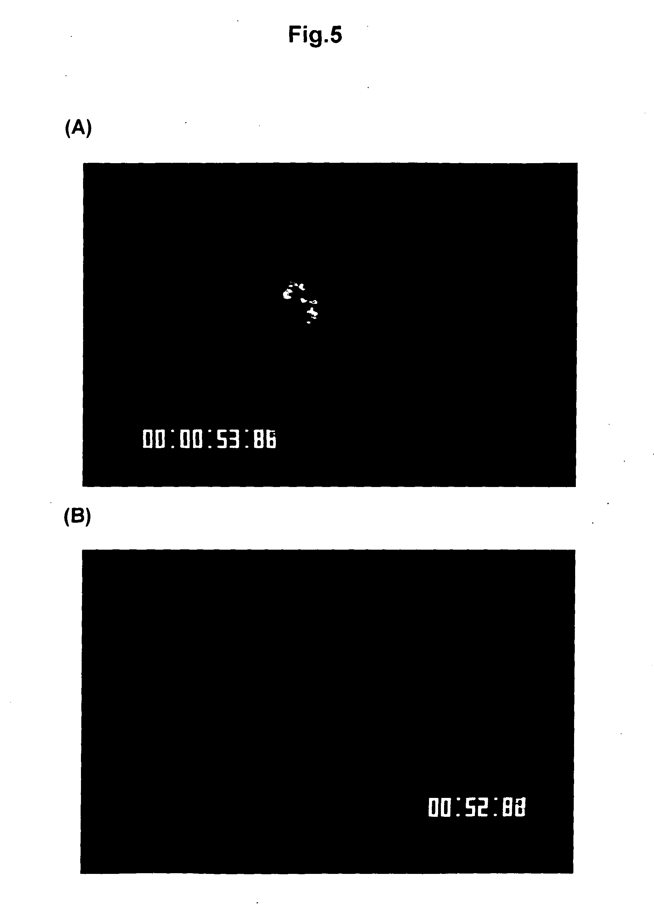

Figure 5 is a view showing differences in formation of microvascular platelet adhesion and aggregation at the site of injury elicited by a laser ablation technique. Figure 5(A) shows the vein of a control, and Figure 5(B) shows the vein of a rat treated with PEG-rHSA. Digit numbers in Figure 5 indicate time after the onset of laser ablation (sec). In the both cases, post-capillary venules (30-50 micron in diameter) were chosen for the analyses in the mesenteric microcirculation. Magenta color indicates accumulation of rhodamine-labeled liposome at the site of the laser-induced injury, the intensity of the labeling being comparable between the control and PEG-rHSA-treated groups. Note that a control venule (Upper panel) exhibits accumulation of CFSE-stained platelets at the site of the injury, while a venule of the rat treated with PEG-rHSA displayed no notable accumulation of the platelets, indicating an anti-thrombotic effect of PEG-rHSA.

### BEST MODE FOR CARRYING OUT THE INVENTION

Hereinafter, the present invention will be described in detail. It should be noted that the scope of the present invention is not limited to the following description and that modifications to the embodiments disclosed herein may be made appropriately without departure of the spirit of the invention. This specification includes all of the contents as disclosed in the specification and/or drawings of US Provisional Patent Application No. 60/778,489, which is a priority document of the present application. All documents and publications cited herein are incorporated herein by reference in their entirety.

### 1. Summary of the present invention

As a result of intensive studies, the present inventors have developed a novel pharmaceutical composition used in an anti-thrombotic treatment, the treatment of an ischemic disease, or the like. In a preferred embodiment of the present invention, the pharmaceutical composition can be a novel anti-thrombotic reagent that differs from those belonging to the aforementioned category.

The pharmaceutical composition of the present invention comprises an albumin conjugated with polyethylene glycol (PEG) (PEG-conjugated albumin). An example of the PEG-conjugated albumin is a "PEG-conjugated human serum albumin" (PEG-HSA), and a particular example thereof is a "PEG-conjugated recombinant human serum albumin (rHSA)" (PEG-rHSA).

When a PEG-conjugated albumin such as PEG-HSA or PEG-rHSA is administered into blood, it increases the viscosity of the blood. It has been known that such increase in the viscosity of the blood increases a wall shear rate, thereby secondarily inducing nitrogen monoxide (NO) *in vivo.* Thus, in a preferred embodiment of the present invention, the pharmaceutical composition can be used as an anti-thrombotic reagent or a reagent for treating an ischemic disease, and it has a function of controlling blood coagulation and/or platelet aggregation in a vascular injury area and the like and exhibits an anti-thrombotic effect and the like.

In previous studies, an attempt has been made to apply PEG-rHSA to the control of hemorrhagic shock (References 1 and 2). However, it has not been known that a PEG-conjugated albumin such as PEG-rHSA has a function of controlling blood coagulation and/or platelet aggregation in a vascular injury area and the like because of a mechanism regarding redistribution and accumulation of circulating platelets under *in vivo* flow condition.

Moreover, the present invention also encompasses: a method of treating a thrombosis or an ischemic disease, comprising intravascularly administering an albumin conjugated with polyethylene glycol to a patient; use of an albumin-conjugated with polyethylene glycol for production of a pharmaceutical composition used in the treatment of an ischemic disease; and use of an albumin conjugated with polyethylene glycol for production of an anti-thrombotic pharmaceutical composition.

### 2. Pharmaceutical composition

### 2.1 Polyethylene glycol-conjugated albumin

The "polyethylene glycol-conjugated albumin" used in the present invention is formed by allowing polyethylene glycol to bind to an albumin. The number of molecules of polyethylene glycol allowed to bind to a single molecule of albumin is, for example, 1 to 20, and preferably 8 to 15.

The type of the polyethylene glycol used in the present invention is not particularly limited. It is preferably an aqueous, biocompatible liquid. The molecular weight of the polyethylene glycol used in the present invention is preferably within the range of 100 to 20,000 Daltons (Da), and more preferably within the range of 1,000 to 10,000 Daltons (Da). Such polyethylene glycol can be easily produced according to known techniques, or commercially available products can be purchased.

Examples of the albumin used in the present invention include: animal albumins such as an egg albumin, a serum albumin, a lactalbumin and a myoalbumin; and vegetable albumins such as leucosin, legumelin and ricin. In particular, it is preferable to use a human serum albumin in the present invention. In addition, a recombinant human serum albumin may also be used. Such a recombinant human serum albumin may have an amino acid sequence identical to that of a human serum albumin, or it may also be a recombinant albumin comprising a deletion, substitution or addition of one or several amino acids. Such an albumin may be easily produced according to known techniques, or commercially available products may be purchased.

The amount of the polyethylene glycol-conjugated albumin contained in the pharmaceutical composition of the present invention is not particularly limited. It can be appropriately determined by persons skilled in the art.

The present invention includes use of the polyethylene glycol-conjugated albumin for production of an anti-thrombotic pharmaceutical composition.

### 2.2 Additives

Moreover, the composition of the present invention may comprise pharmaceutically acceptable additives for the purposes of: (i) the improvement of the stability of the drug in a polymeric composition; (ii) the control of the concentration of hydrogen ion in the composition; (iii) the control of the release rate of the drug and the decomposition rate of polymer; etc. Such additives are preferably selected from the group consisting of surfactants, inorganic salts, sugars, natural polymers and mixtures thereof. Either ionic or non-ionic surfactants may be used in the composition of the present invention. Typical ionic surfactants include sodium dodecylsulfate and typical non-ionic surfactants include polysorbate, polyvinylpyrrolidone, poloxamers, glyceryl monooleate, glyceryl monostearate, polyoxyethylene alkyl ether and mixtures thereof.

The sugars which can be used as additives in the present invention include mannitol, sorbitol, glucose, xylitol, trehalose, sorbose, sucrose, galactose, dextran, dextrose, fructose, lactose and mixtures thereof.

Examples of inorganic salts which can be used as additives in the present composition include sodium chloride, calcium chloride, zinc chloride, magnesium chloride, calcium carbonate, zinc carbonate, zinc acetate, zinc lactate, magnesium hydroxide, aluminum chloride, aluminum hydroxide, zinc oxide and mixtures thereof.

Examples of natural polymer which can be used as additives in the present composition include cyclodextrin, gelatin, hyaluronic acid, chitosan, sodium carboxymethylcellulose and mixtures thereof.

The content of additives in the composition of the present invention is preferably within the range of 0.01 to 10 wt %, and more preferably within the range of 0.1 to 1 wt %.

### 2.3 Administration of pharmaceutical composition

In a preferred embodiment of the present invention, by administering the pharmaceutical composition into a living body, blood coagulation and/or platelet aggregation can be controlled in a vascular injury area, so that formation of thrombus can be effectively prevented. Thus, the pharmaceutical composition of the present invention is useful for an anti-thrombotic treatment or the treatment of an ischemic disease.

The administration route of the pharmaceutical composition of the present invention is not particularly limited. For example, a parenteral administration is preferable. In the case of such a parenteral administration, the pharmaceutical composition is preferably administered in the form of a liquid preparation such as an injection. In the present invention, such an injection is particularly preferably a drip injection. When the pharmaceutical composition is administered in the form of an injection, it is administered as an intravenous injection, a subcutaneous injection, an intracutaneous injection, an intramuscular injection, etc. The dose of the pharmaceutical composition of the present invention is different depending on the age, sex and symptoms of a patient, an administration route, the number of doses, and a dosage form. Thus, the dose of the present pharmaceutical composition is not particularly limited, and it can be selected, as appropriate. For example, the dose is appropriately selected by a clinician.

### 3. Method of producing pharmaceutical composition

A method of producing the composition of the present invention is not particularly limited, and a known production method can be applied. For example, when the pharmaceutical composition of the present invention is produced in the form of an injection, it is prepared by dissolving, suspending or emulsifying an albumin conjugated with polyethylene glycol in a sterile aqueous or oily liquid. Such an aqueous liquid used for injection includes a normal saline solution, an isotonic solution containing glucose or other auxiliary agents (e.g. D-sorbitol, D-mannitol, sodium chloride, etc.), and the like. Suitable solubilizing agents such as alcohol (e.g. ethanol, etc.), polyalcohol (e.g. propylene glycol, polyethylene glycol, etc.), and a nonionic surfactant (e.g. polysorbate 80, HCO-50, etc.) may be used together with such an aqueous liquid. On the other hand, an oily liquid includes sesame oil and soybean oil. Solubilizing agents such as benzyl benzoate and benzyl alcohol may be used together with such an oily liquid. In addition, such an oily liquid may also be mixed with a buffer (e.g. a phosphate buffer, a sodium acetate buffer, etc.), a soothing agent (e.g. benzalkonium chloride, procaine hydrochloride, etc.), a stabilizer (e.g. polyethylene glycol, etc.), a preservative (e.g. benzyl alcohol, phenol, etc.), and the like. The prepared injection is generally filled into an ampule.

Moreover, the polyethylene glycol-conjugated albumin used in the present invention can be obtained by allowing polyethylene glycol (PEG) to react with an albumin (e.g. a human serum albumin (HSA) or a recombinant human serum albumin (rHSA)). Specifically, the polyethylene glycol-conjugated albumin solution of the present invention can be produced by a method which comprises a step of mixing and dissolving a water-soluble biocompatible liquid polyethylene glycol and an albumin (e.g. HSA, rHSA, etc.) as a physiologically active substance in an organic solvent or in a mixture of an organic solvent and water to obtain a polymeric solution. Furthermore, such a polymeric solution is filtrated by passing it through a membrane for sterilization, and the thus filtrated polymeric solution is then freeze-dried or evaporated under reduced pressure, so that the organic solvent may be eliminated.

More specifically, the polyethylene glycol-conjugated albumin solution of the present invention can be produced by dissolving a liquid polyethylene glycol, an albumin (e.g. HSA, rHSA, etc.), and any given additives in an organic solvent or in a mixture of an organic solvent and water. Examples of an organic solvent suitable for preparing the solution include acetonitrile, acetone, acetic acid, dimethyl acetamide, ethanol, isopropanol, and dioxane.

### 4. Therapeutic method

The present invention also provides a method of treating a thrombosis or an ischemic disease, which comprises intravascularly administering a polyethylene glycol-conjugated albumin. Targets to be treated are not particularly limited. Examples of such a therapeutic target include a human, and organisms other than a human including nonhuman mammals such as a bovine, a simian, an Aves, a feline, a mouse, a rat, a guinea pig, a hamster, a swine, a canine, a rabbit, a sheep or a horse.

Herein, it is considered that the therapeutic method of the present invention functions by taking advantage of the action of the albumin molecule during an ischemic ("oxygen-depleted") event. It is to be noted that the term "ischemic event" is used in the present specification to mean that the patient has experienced a local and/or temporary ischemia due to insufficient blood flow (partial or total obstruction of the blood circulation) to an organ. Moreover, the term "anti-thrombotic treatment" is used herein to mean a treatment for suppressing thrombus formation.

Furthermore, the term "ischemic disease" is used to mean a disease that causes such an ischemic event. Examples of such an ischemic disease include external injuries, immunological rejection occurring during transplantation, ischemic cerebral vascular accident (e.g. apoplectic stroke, cerebral infarction, etc.), ischemic renal disease, ischemic pulmonary disease, ischemic disease associated with infectious disease, ischemic disease of limbs (e.g. arteriosclerosis obliterans), and ischemic heart disease (e.g. ischemic cardiomyopathy, myocardial infarction, ischemic heart failure, etc.). Other examples also include diseases that cause ischemic events, such as cerebral edema and acute coronary failure.

Preferably, a polyethylene glycol-conjugated albumin is intravascularly administered in the form of a liquid preparation such as an injection. In addition, in a preferred embodiment of the present invention, such a polyethylene glycol-conjugated albumin is intravenously administered.

In addition, the present invention also includes use of a polyethylene glycol-conjugated albumin for production of a pharmaceutical composition used in the treatment of a human ischemic disease.

Hereinafter, the present invention will be described in more detail with reference to the following Example. However, the present invention is not limited to this Example.

### [EXAMPLE 1]

### 1 Materials and Methods

### 1.1 Animals

Male Wistar rats weighing 230-290 g were overnight fasted under free access of water and anesthetized with isoflurane inhalation according to the previous methods (reference 3). Bleeding times were determined using a method previously described (reference 4). A 2.5 mm long 1 mm deep template-guided incision was made in the tails of anesthetized rats, 1 cm from the tip of the tail; 45 degrees from the dorsal vein. Tails were immersed in 45 ml of normal saline, and the time until bleeding stopped was measured with a stopwatch.

### 1.2 Preparation of PEG-rHSA

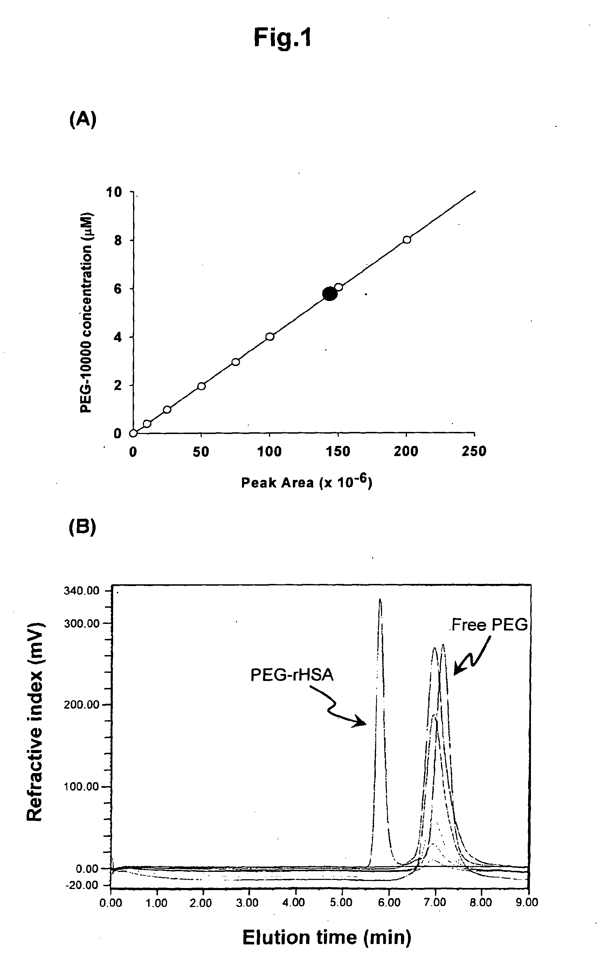

Recombinant human serum albumin (rHSA) is a generous gift from NIPRO Inc (Shiga, Japan). Polyethylene glycol (PEG) with a molecular weight of 10,000 was commercially available. PEG-bound rHSA was prepared by reacting rHSA with succinimidyl-PEG precursor which has the ability to react with lysine residue of the protein, according to previous methods (reference 5). The number of PEG bound per a molecule of rHSA was calculated by determining free PEG in the reaction mixture for the PEG-rHSA preparation using high-performance liquid chromatography (HPLC, TSKgel column with Waters 2695 separation module). In order to quantify the free unbound PEG in the reaction mixture, known concentrations of PEG were prepared for establishment of the calibration line. The amounts of PEG were determined by calculating the areas of the peak of HPLC charts. As seen in Figure 1, the calculation data indicated that approximately 10-11 molecules of PEG were bound to one molecule of rHSA.

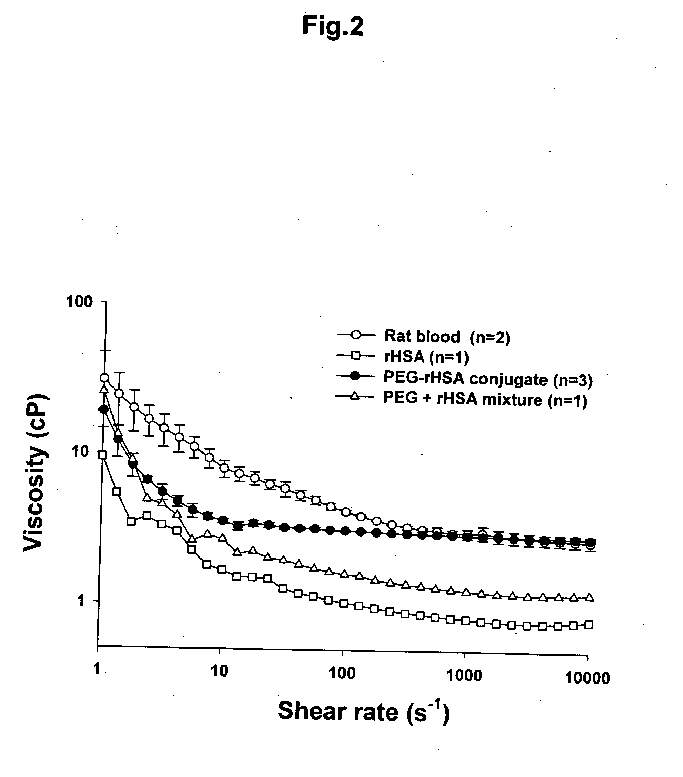

The effect of PEG-rHSA on the blood viscosity was examined *in vitro* using a rotation fluid viscometer. Parameters for characterization of the samples were shown in Table 1. As shown in Figure 2, PEG-rHSA exhibited a viscosity comparable to the whole blood sample alone (•), while the mixture with PEG-unbound rHSA caused a marked reduction of the viscosity versus the whole blood (△). On the other hand, rHSA alone displayed the smallest value of the viscosity (□). Another feature of PEG-rHSA is a greater value of colloid osmotic pressure (COP) in PEG-rHSA than in rHSA (Table 1). These results suggest that PEG-rHSA has the ability to maintain physiological blood viscosity.

### 1.3 Animal model of isovolemic hemodilution

Rats were anesthetized with diethyl ether followed by isoflurane. An arterial catheter was implanted in the left femoral artery, connected to a pressure transducer, and mean arterial pressure (MAP) was amplified and monitored continuously, and the data was collected on a computer. The right femoral vein was cannulated for infusion of solutions and the right femoral artery was cannulated for withdrawing blood and sampling arterial blood for the blood gas analysis, hematocrit and platelet count. Samples were taken in EDTA tubes twice before and after the hemodilution (at the cannulation and the end of the observation period). Rectal temperature was monitored and kept stably from the start of monitoring.

After three catheter implantations, the stability of MAP was observed in 5 minutes. Each solution (rHSA, PEG-rHSA conjugate, PEG+rHSA mixture) was infused with a dual syringe pump into the right femoral vein catheter in 5 minutes, and blood was simultaneously withdrawn from the right femoral artery catheter at the same rate. The volume of exchange transfusion was calculated as a 30% of the rat's blood volume, estimated as 56ml/kg of body weight for rats. The rate of exchange transfusion was calculated as: (The volume of exchange transfusion) / 5 [ml/min]

At 10 min after initiation of the blood exchange, the position of the rat was changed and prepared for the tail cut. At 15 min after initiation of the blood exchange, in five minutes after the position-change, a template-guided incision (Quikheel, Becton-Dickinson, San Jose, CA) 2.5 mm in length and 1.0 mm in depth was made 1 cm from the tip of the tail. The tail was immersed in a 50 ml cylinder of saline, and the time taken for bleeding to stop was measured. The monitoring of MAP was continued until 50 min, when the second sample was collected.

**Table 1.**

| **Characteristics of the solutions studied** | | |
|---|---|---|
| | rHSA | PEG-rHSA |
| rHSA, g/l | 13.4 | 13.4 |
| PEG [M] / rHSA [M] | -- | 11∼12 |
| Viscosity at 562 /s, cPs | 0.85 | 3.00 |
| COP, mmHg | 3.8 | 58.6 |
| Solvent | Saline | Saline |

### 1.4 Intravital observation of platelet adhesion and aggregation in vivo using laser ablation.

In separate sets of experiments, differences in platelet adhesion and aggregation at the site of vascular injury between the group treated with PEG-rHSA and that treated with free rHSA were compared using an *in vivo* model of microvascular laser ablation according to a known method (reference 6). In brief, rats were injected with carboxy fluorescein diacetate succinimidyl ester (CSFE), a fluorescent precursor that can stain circulating platelets and partly leukocytes when injected into circulation (reference 7). After the CFSE injection, 200 ml of PEG-coated liposome labeled with rhodamine G was injected. This liposomal particle does not accumulate nor adhere to the intact vascular wall, but accumulate quickly to the subendothelial space of the injured vascular endothelial cells.

To elicit microvascular thrombosis, the inventors used a multi-pinhole confocal laser microscope (Yokokawa Medical Inc., Tokyo) equipped with a nitrogen-dye pulse laser system that allows the inventors to carry out pin-point laser ablation of the microvascular endothelial cells located at the area of interests. The energy of the laser ablation was set constant among different experiments. When the pulse laser was applied to a microvessel chosen for the analysis, the damaged endothelial cells became noticeable through accumulation of the rhodamine-labeled liposome which can be visualized as red color (or magenta) under excitation with 530 nm. On the other hand, alterations in platelet adhesion and aggregation were visualized under epi-illumination at 488 nm as green color. Using a wide-range band-pass filter, both green and red fluorescent signals at the site of the vascular injury were visualized simultaneously through intensified CCD camera (Hamamatsu Photonics Inc., Hamamatsu City) and a digital video recording system.

### 1.5 Statistical analysis

Values are presented as means±S.E. unless otherwise indicated. Statistical significance is reported at the P<0.05.

### 2. Results and Discussion

### 2.1 Improving effects of PEG-rHSA on MAP and heart rates during 30% isovolemic hemodilution procedure.

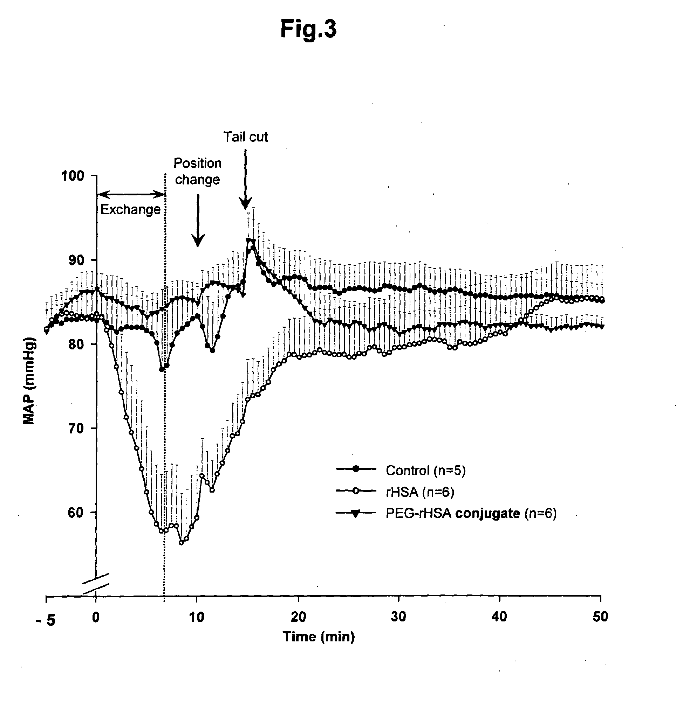

Figure 3 illustrates time history of MAP during the blood exchange procedure for 30% isovolemic hemodilution, respectively. As shown in Figure 3, the group treated with rHSA alone exhibited a significant reduction of MAP versus the control untreated with the hemodilution during the period for the blood exchange. The MAP values exhibited a recovery after completing the exchanging procedure, but were not fully recovered up to the control level. On the other hand, the group treated with PEG-rHSA did not display any notable reduction of MAP throughout the whole course of observation. The group treated with rHSA plus free unbound PEG at a comparable dose also exhibited no remarkable reduction of the MAP values, but such an effect appeared to be smaller than that observed in the group treated with PEG-rHSA.

### 2.2 Administration of PEG-rHSA elongates bleeding time in vivo.

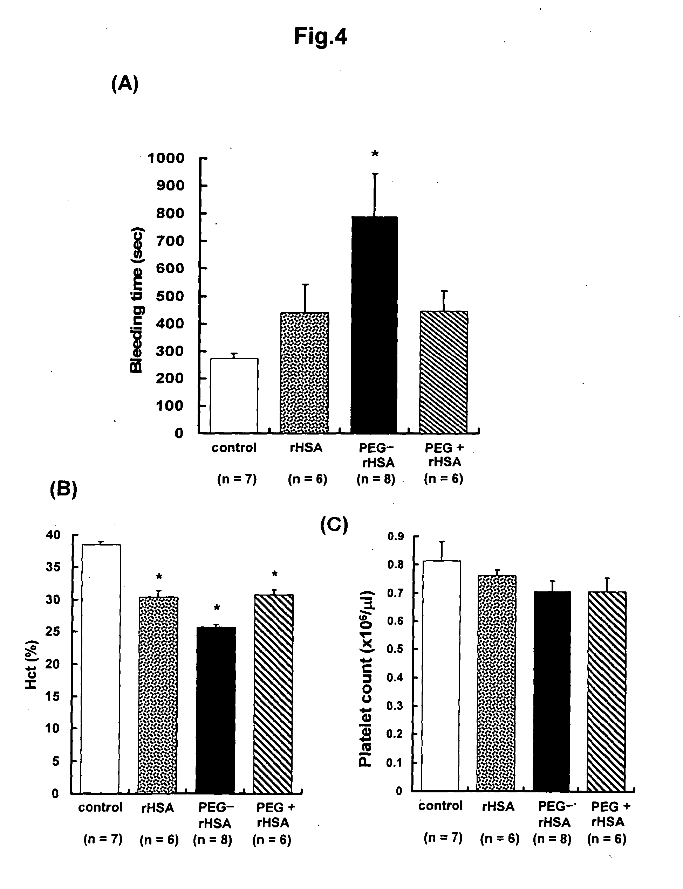

Figure 4 showed differences in bleeding time measured by the tail cut procedure among the groups. As shown in Figure 4(A), the group treated with PEG-rHSA caused a significant elongation of hemorrhagic time versus the group treated with rHSA alone or with rHSA plus free PEG. These results led the inventors to examine changes in circulating platelet counts and hematocrit values among the groups. As shown in Figure 4(B), in the rats treated with a conjugate of PEG and rHSA (PEG-rHSA), with rHSA, or with a mixture of rHSA and free PEG (PEG+rHSA), the hematocrit values were decreased by 25-30% almost comparably as a result of hemodilution. Among these three groups, the group treated with PEG-rHSA exhibited the smallest value of hematocrit presumably because of the ability to maintain water molecules in an intravascular space. Of interest is that circulating platelet counts were not greatly reduced as compared with the reduction of hematocrit values caused by hemodilution in these groups (Figure 4(C)). As a consequence, the platelet counts versus plasma compartment (Platelets / 100-Hct %) became smallest in the group treated with PEG-rHSA. These results tempted the inventors to examine efficiency of platelet recruitment at the site of injury *in vivo* using microvascular laser ablation technique as follows.

### 2.3 Administration of PEG-rHSA attenuates laser-induced microvascular thrombogenesis in vivo.

Based on results shown in Figure 4, the inventors hypothesized that, under isovolemic hemodiluted conditions, platelets marginating in and around microvascular endothelium could shift their distribution to centerline regions of the vasculature upon the administration of PEG-rHSA, and it was thereby assumed that apparent counts of circulating platelets were elevated in spite of a reduction of the cells resulting from the blood-exchanging procedure.

Since the systemic blood pressure was slightly higher in the PEG-rHSA-treated group than in rHSA-treated group, the inventors chose post-capillary venules where the local perfusion pressure would be comparable between the groups. As seen in Figure 5, magenta color indicates accumulation of rhodamine-labeled liposome at the site of the laser-induced injury, the intensity of the labeling being comparable between the control and PEG-rHSA-treated groups. On the other hand, the magnitude of the platelet adhesion and aggregation appeared to be different. As shown in Figure 5(A), a control venule exhibits accumulation of CFSE-stained platelets at the site of the injury, while a venule of the rat treated with PEG-rHSA as shown in Figure 5(B) displayed no notable accumulation of the platelets, indicating an anti-thrombotic effect of PEG-rHSA. Accordingly, it was found that a polyethylene glycol-conjugated albumin can be used in an anti-thrombotic treatment or the treatment of an ischemic disease.

### INDUSTRIAL APPLICABILITY

PEG-rHSA has the remarkable effect to maintain retention of water into an intravascular space and helps improvement of microvascular hemodynamics with antiplatelet properties. Thus, the PEG-rHSA administration is likely to be beneficial to treat patients with acute ischemic stroke of cerebral microcirculation and brain edema or with acute coronary insufficiency.

### REFERENCES

1. Hangai-Hoger N, Nacharaju P, Manjula BN, Cabrales P, Tsai AG, Acharya SA, Intaglietta M. Microvascular effects following treatment with polyethylene glycol-albumin in lipopolysaccharide-induced endotoxemia. Crit Care Med. 2006, 34, 108-117.
2. Cabrales P, Nacharaju P, Manjula BN, Tsai AG, Acharya SA, Intaglietta M. Early difference in tissue pH and microvascular hemodynamics in hemorrhagic shock resuscitation using polyethylene glycol-albumin- and hydroxyethyl starch-based plasma expanders. Shock. 2005 Jul;24(1):66-73
3. Kimbro JR, Kelly PJ, Drummond JC, Cole DJ, Patel PM. Isoflurane and pentobarbital reduce AMPA toxicity in vivoin the rat cerebral cortex. Anesthesiology. 2000 Mar;92(3):806-12.
4. RYAN J. HANSEN and JOSEPH P. BALTHASAR Pharmacokinetics, Pharmacodynamics, and Platelet Binding of an Anti-Glycoprotein IIb/IIIa Monoclonal Antibody (7E3) in the Rat: A Quantitative Rat Model of Immune Thrombocytopenic Purpura, THE JOURNAL OF PHARMACOLOGY AND EXPERIMENTAL THERAPEUTICS, 2001, 298: 165-171.
5. Ito T, Fujihara M, Abe H, Yamaguchi M, Wakamoto S, Takeoka S, Sakai H, Tsuchida E, Ikeda H, Ikebuchi K. Effects of poly(ethyleneglycol)-modified hemoglobin vesicles on N-formyl-methionyl-leucyl-phenylalanine-induced responses of polymorphonuclear neutrophils in vitro. Artif Cells Blood Substit Immobil Biotechnol. 2001 Nov;29(6):427-37.
6. Falati S, Gross P, Merrill-Skoloff G, Furie BC, Furie B Real-time in vivo imaging of platelets, tissue factor and fibrin during arterial thrombus formation in the mouse Nat Med. 2002 Oct;8(10):1175-81. Epub 2002 Sep 16.
7. Katayama T., Ikeda Y., Handa M., Tamatani T., Sakamoto S., Ito M., Ishimura Y., Suematsu M. Immunoneutralization of glycoprotein Ibalpha attenuates endotoxin-induced interactions of platelets and leukocytes with rat venular endothelium in vivo, Cir Res 2000 86 (10): 1031-1037

## Claims

1. A pharmaceutical composition comprising an albumin conjugated with polyethylene glycol.

2. The composition according to claim 1, which is an anti-thrombotic reagent or a reagent for treating an ischemic disease.

3. The composition according to claim 1 or 2, wherein said albumin is a human albumin.

4. The composition according to claim 1 or 2, wherein said albumin is a recombinant human serum albumin.

5. A method of treating a thrombosis or an ischemic disease, comprising intravascularly administering an albumin conjugated with polyethylene glycol to a patient.

6. The method according to claim 5, which treats a human.

7. The method according to claim 5 or 6, wherein said intravascular administration is an intravenous administration.

8. The method according to any one of claims 5 to 7, wherein said albumin is a human albumin.

9. The method according to any one of claims 5 to 7, wherein said albumin is a recombinant human serum albumin.

10. Use of an albumin conjugated with polyethylene glycol for production of a pharmaceutical composition used in a treatment of an ischemic disease

11. Use of an albumin conjugated with polyethylene glycol for production of an anti-thrombotic pharmaceutical composition.
